# EUROPEAN PATENT SPECIFICATION

(11) **EP 2 279 162 B1**
(45) Date of publication and mention of the grant of the patent: **04.01.2012**
(21) Application number: 09745705.5
(22) Date of filing: 11.05.2009
(51) Int. Cl.: C07C 67/08, C07C 69/743, C07C 253/30

(54) **PROCESSES FOR THE PREPARATION OF ESTERS**
VERFAHREN ZUR HERSTELLUNG VON ESTERN
PROCÉDÉS DE PRÉPARATION D'ESTERS

(30) Priority: 14.05.2008 GB 0808767
(43) Date of publication of application: 02.02.2011
(73) Proprietor: Syngenta Limited, Guildford Surrey GU2 7YH (GB); Syngenta Participations AG, 4058 Basel (CH)
(72) Inventor: BOWDEN, Martin, Charles, Bracknell Berkshire RG42 6EY (GB); JACKSON, David, Anthony, CH-4333 Münchwilen (CH); SAINT-DIZIER, Alexandre, Christian, CH-1870 Monthey (CH)
(74) Representative: Thwaite, Jonathan Simon
(86) International application number: PCT/EP2009/055649
(87) International publication number: WO 2009/138373

(56) References cited:
- EP-A- 0 067 461
- EP-A- 0 310 954
- EP-A- 1 227 077
- EP-A- 1 547 995
- EP-A- 1 792 889
- DATABASE WPI Thomson Scientific, London, GB; AN 1997-140863 XP002559359 & JP 09 020718 A (SUMITOMO CHEM CO LTD) 21 January 1997 (1997-01-21)

## Description

The present invention relates to a process for the preparation of certain pyrethroid compounds useful as insecticides, as well as a process for the preparation of intermediates useful in the synthesis of such compounds.

Synthetic pyrethroid insecticides of the formula (I) are disclosed in GB2000764 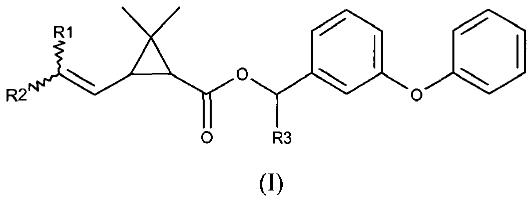 wherein
one of R1 and R2 represents a group of formula: W~(CF₂)m~ where W represents an atom of hydrogen, fluorine or chlorine and m has the value one or two, and the other of R1 and R2 represents an atom of fluorine, chlorine or bromine, and R3 represents an atom of hydrogen or the cyano or ethynyl group.

Synthetic pyrethroid insecticides of the formula (II) are disclosed in US4405640 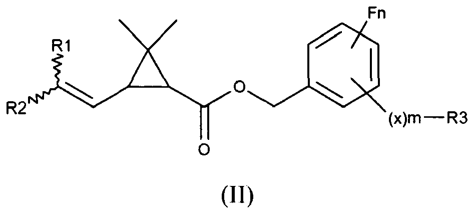 wherein
R1 and R2 are each selected from methyl, halomethyl, and halo; X is oxygen, sulphur, sulphonyl or a group NR4 where R4 represents hydrogen, lower alkyl or lower carboxylic acyl; R3 is lower alkyl, lower alkenyl or benzyl; m has the value zero to one, and n has a value from one to four.

EP-A-0 067 461 discloses the preparation of synthetic pyrethroid insecticide esters by reacting a cyanobenzyl arylsulphonate with a cyclopropane carboxylic acid.

JP-A- 9 020 718 discloses the preparation of phenoxy-benzyl ester derivatives useful as insecticides by reacting phenoxy-benzyl alcohol with carboxylic chloride in presence of a base and a phase transfer catalyst.

EP-A-0 310 954 discloses the preparation of ortho-substituted benzyl carboxylates by reacting an ortho-substituted benzyl bromide with an alkali metal salt, alkaline earth metal salt or ammonium salt of a carboxylate.

The present invention seeks to provide processes for the preparation of these and other pyrethroids.

It has now surprisingly been found that these compounds may advantageously obtained by coupling the corresponding alcohol and carboxylic acid in the presence of a catalyst, as described below.

The present invention provides a process for the preparation of compounds of formula (III) 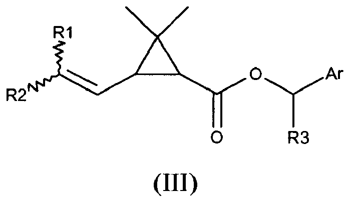 wherein
R1 is hydrogen, alkyl, haloalkyl, or halogen;
R2 is hydrogen, alkyl, haloalkyl, or halogen;
R3 is hydrogen, halogen, hydroxyl, nitro, cyano, optionally substituted alkyl, optionally substituted alkenyl, or optionally substituted alkynyl;
Ar is an optionally substituted aryl group
comprising reacting a carboxylic acid of formula (IV) 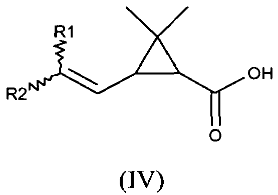 with an alcohol of the formula (V) 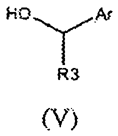 in the presence of a catalyst, selected from ammonium sals catalysts.

Preferably, R2 and R2 are independently selected from hydrogen, halogen, C1-C4 alkyl and C1-C4 haloalkyl.

Preferably, R3 is reeled from hydrogen, halogen, hydroxyl, nitro, cyano, C1-C4 alkyl and C1-C4 haloalkyl, more preferably hydrogen, cyano or C3-C4 alkynyl.

Preferably Ar is phenyl substituted with one or more groups selected from hydrogen, halogen, hydroxyl, nitro, cyano, C1-C4 alkyl, C1-C4 haloalkyl, and phenoxy.

Halo is fiuoro, chloro or bromo.

Each alkyl moiety is a straight or branched chain and is, for example, methyl, ethyl, n-propyl, n-butyl, n-pentyl, n-hexyl, iso-propyl, n-butyl, sec-butyl, iso-butyl, tert-butyl or neo-pentyl. Preferred alkyl groups have from 1 to 6 carbon atoms.

Haloalkyl refers to an alkyl moiety as defined above wherein at least one hydrogen atom is substituted for a halo atom.

Acryl refers to a phenyl or napthyl group.

Heteroaryl refers to a mono- or bicyclic ring system wherein each ring comprises from 5 to 7 ring member atoms, and from 1 to 3 heteroatoms independently selected from O, N and S. Examples of heteroaryl groups are pyridine, pyrrole, furan, pyrazole, imidazole and oxazole.

Alkenyl refers to a straight or branched group consisting of carbon and hydrogen atoms comprising at least one carbon-carbon double bond. Examples are ethenyl, prop-1-enyl, prop-2-enyl, but-1-enyl, but-2-enyl and but-3-enyl. Both *cis*- and *trans*- groups are contemplated. Preferred alkenyl groups have from 2 to 6 carbon atoms.

Alkynyl refers to a straight or branched group consisting of carbon and hydrogen atoms comprising at least one carbon-carbon triple bond. Examples are ethynyl, prop-1-ynyl, prop-2-ynyl, but-1-ynyl, but-2-ynyl and but-3-ynyl. Preferred alkynyl groups have from 2 to 6 carbon atoms.

Cycloalkyl includes cyclopropyl, cyclobutyl, cyclopentyl and cyclohexyl.

When present, each optional substituent on aryl or on heteroaryl is, independently, preferably selected from alkyl, alkenyl, alykynyl, alkoxyl, alkenyloxy, aryloxy, thioalkyl, amino, alkylamino, dialkylamino, aralkyl, acetamido, n-alkyl acetamido, alkylsufonyl, halo, hydroxyl, cyano and nitro. From one to the maximum possible number of substituents may be present.

Preferably, one of R1 and R2 is halo, and the other is haloalkyl. More preferably, one of R1 and R2 is chloro, and the other is haloalkyl, e.g. halo-C -C4-alkyl. More preferably, one of R1 and R2 is halo, and the other is trifluoromethyl. Most preferably, one of R1 and R2 is chloro, and the other is trifluoromethyl.

Preferably, the substituents on the cyclopropane ring are in the *cis*- stereochemistry. That is, the compound of formula (III) has the stereochemistry (IIIa)

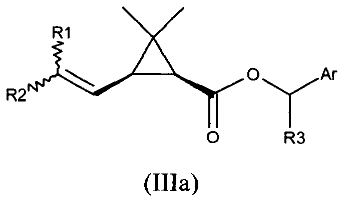

Preferably, the compound of formula (III) has the formula (IIIb)

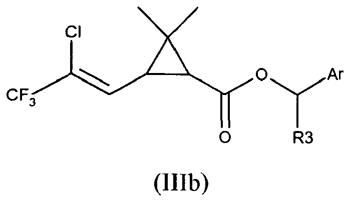

Very preferably, the compound of formula (III) has the formula (IIIc)

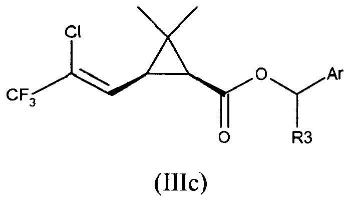

In a preferred embodiment, Ar is an optionally substituted phenyl group.

In a highly preferred embodiment, Ar is a phenoxyphenyl group. More preferably, Ar is a 3-phenoxyphenyl group. In this embodiment, it is preferred that R3 is cyano or ethynyl. It is more preferred that R3 is cyano.

For example,
One of R1 and R2 is halo, and the other is halo-C1-C4-alkyl;
R3 is cyano or ethynyl;
Ar is is a phenoxyphenyl group.

Very highly preferably, the compound of formula (III) has the formula (IIId)

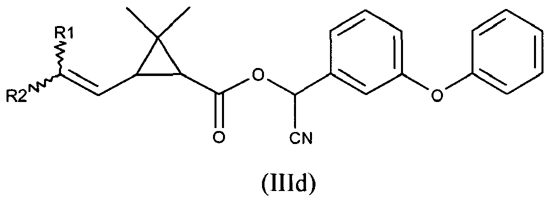

In an alternative preferred embodiment, Ar is a group of the formula (VI) 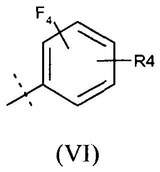 wherein R4 is selected from alkyl, alkenyl, alykynyl, alkoxyl, alkenyloxy, aryloxy, thioalkyl, amino, alkylamino, dialkylamino, aralkyl, acetamido, n-alkyl acetamido, alkylsufonyl, halo, hydroxyl, cyano and nitro. Preferably, R4 is alkyl of 1 to 4 carbon atoms, alkenyl of three to five carbon atoms, methoxy, ethoxy, allyloxy, ethylthio, ethanesulphonyl, benzyl, dimethylamino, ethylamino, acetamido or n-methylacetamido. Very preferably, R4 is methyl. Preferably, R4 is in the 4-position.

In this embodiment, it is preferred that R3 is hydrogen.

For example,
One of R1 and R2 is halo, and the other is halo-C 1-C4-alkyl;
R3 is hydrogen;
Ar is a group of the formula (VI) 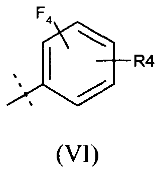 R4 is alkyl of I to 4 carbon atoms, alkenyl of three to five carbon atoms, methoxy, ethoxy, allyloxy, ethylthio, ethanesulphonyl, benzyl, dimethylamino, ethylamino, acetamido or n-methylacetamido.

Very highly preferably, the compound of formula (III) has the formula (IIIe)

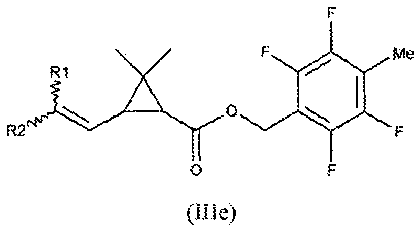

According to a vary highly preferred embodiment, the invention provides a process for the preparation of tefluthrin (VII) 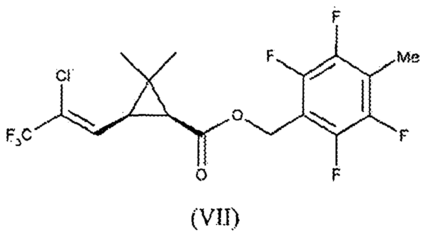 comprising reading a carboxylic acid of formula (VIII) with an alcohol of formula (IX) 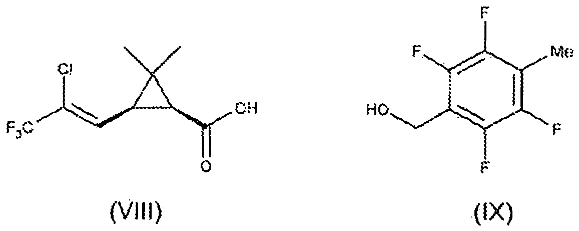 in the presence of a catalyst selected from ammonium salt catalysts.

According to an alternative very highly preferred embodiment, the invention provides a process for the preparation of lambda cyhalothrin (X) 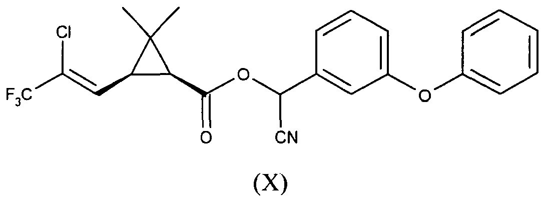 comprising reacting a carboxylic acid of formula (VIII) with an alcohol of formula (XI) 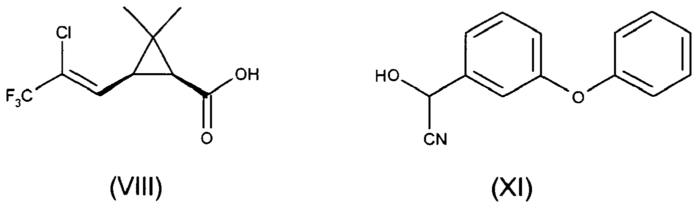 in the presence of a catalyst selected from ammonium salt catalysts.

Compound (XI) may be generated *in situ.*

The present invention also relates to a process substantially as described herein with reference to the examples.

### Enantiomers

The processes of the invention may be used to prepare enantiomerically enriched or pure forms of the compounds. In a preferred embodiment, the invention provides a process for the preparation of gamma-cyhalothrin (S)-a-cyano-3-phenoxybenzyl (Z) - (1R,3R)-3-(2-chloro-3,3,3-trifluoro-1-propenyl)-2,2-dimethylcyclopropane carboxylate) (XIV)

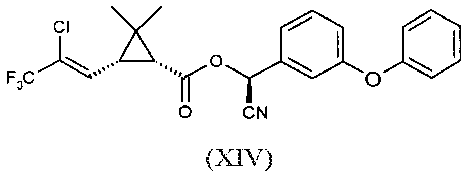

### Solvent

The reaction of the invention is optionally (and preferably) conducted in a suitable solvent. Suitable solvents include, but are not limited to, linear, branched or cyclic aliphatic hydrocarbons, such as ligroin or cyclohexane, pentane, hexane, heptane, octane, as well as aromatic solvents, such as benzene, toluene, xylene, monochlorobenzene, dichlorobenzene, trichlorobenzene.

A preferred solvent is xylene.

### Temperature

The reaction of the invention may be carried out at a temperature such that an acceptable rate of reaction is attained. Preferably, the reaction is conducted at a temperature of from 0 °C to 200 °C. More preferably, the reaction is conducted at a temperature of from 50 °C to 180 °C. More preferably, the reaction is conducted at a temperature of from 100 °C to 170 °C. More preferably, the reaction is conducted at a temperature of from 130 °C to 150 °C.

### Removal of Water

Preferably, provision is made for removal of water from the reaction mixture, e.g. removal of water prior to completion of the reaction. Water may be removed from the reaction continuously. A suitable method is azeotropic removal of water. Suitable apparatus for conducting azeotropic removal of water will be known to those skilled in the art. We have found that removal of water is highly desirable in order to achieved a commercially useful conversion to product.

### Catalyst

A preferred ammonium salt catalyst is Ph₂NH₂OTf.

### Amount of Catalyst

Preferably, the amount of catalyst employed is up to 50 mol % based on the amount of carboxylic acid (IV). More preferably, the amount of catalyst employed is up to 25 mol % based on the amount of carboxylic acid (IV). More preferably, the amount of catalyst employed is up to 15 mol % based on the amount of carboxylic acid (IV).

Preferably, the amount of catalyst employed is at least 0.01 mol % based on the amount of carboxylic acid (IV). More preferably, the amount of catalyst employed is at least 0.1 1 mol % based on the amount of carboxylic acid (IV). More preferably, the amount of catalyst employed is at least 1 mol % based on the amount of carboxylic acid (IV). More preferably, the amount of catalyst employed is at least 5 mol % based on the amount of carboxylic acid (IV)

Preferably, the amount of catalyst employed is between 0.01 and 50 mol % based on the amount of carboxylic acid (IV). More preferably, the amount of catalyst employed is between 0.1 and 25 mol % based on the amount of carboxylic acid (IV). More preferably, the amount of catalyst employed is between 5 and 15 mol % based on the amount of carboxylic acid (IV).

Preferably, the amount of catalyst employed is up to 50 mol % based on the amount of alcohol (V). More preferably, the amount of catalyst employed is up to 25 mol % based on the amount of alcohol (V). More preferably, the amount of catalyst employed is up to 15 mol % based on the amount of alcohol (V).

Preferably, the amount of catalyst employed is at least 0.01 mol % based on the amount of alcohol (V). More preferably, the amount of catalyst employed is at least 0.1 mol % based on the amount of alcohol (V). More preferably, the amount of catalyst employed is at least 1 mol % based on the amount of alcohol (V). More preferably, the amount of catalyst employed is at least 5 mol % based on the amount of alcohol (V).

Preferably, the amount of catalyst employed is between 0.01 and 50 mol % based on the amount of alcohol (V). More preferably, the amount of catalyst employed is between 0. 1 and 25 mol % based on the amount of alcohol (V). More preferably, the amount of catalyst employed is between 5 and 15 mol % based on the amount of alcohol (V).

### Synthesis of Starting Materials

Suitable methods for the preparation of carboxylic acids (IV) and alcohols (V) are disclosed in US4405640 and GB2000764. Other methods will be apparent to those skilled in the art.

### Workup and Isolation of Products

Workup of the reaction mixture is achieved according to well known procedures of synthetic organic chemistry. For example, an aqueous workup may be achieved by the addition of water (or other aqueous solution), and extraction of the desired product with a suitable organic solvent.

Alternatively, the product may be isolated by removing any solvent present by distillation, e.g. under reduced pressure.

Purification of the product may be achieved by any one of a number of methods, e.g. distillation, recrystallization and chromatography.

The present invention will now be described by way of the following non-limiting examples. Those skilled in the art will promptly recognize appropriate variations from the procedures both as to reactants and as to reaction conditions and techniques.

All aspects and preferred features of the invention may be combined with each other, except where this is evidently not possible.

### Examples

### Reference Example 1

### Reaction Sequence

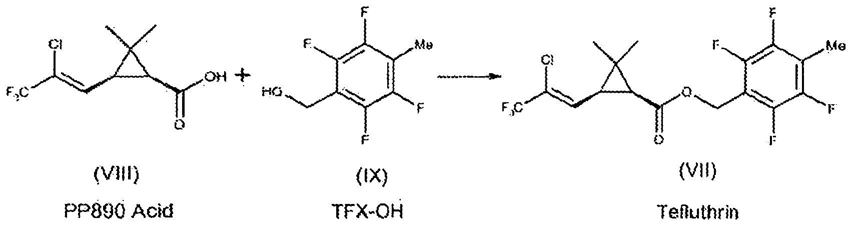

| Solvent | Conditions | Time | Catalyst | Conversion |
|---|---|---|---|---|
| Xylene | Reflux, Dean & Stark | 3 hours | ZrCl₄ (1 Mol %) | 2 % |
| Xylene | Reflux, Dean & Stark | 3 hours | ZrCl₄ (10 Mol %) | 100% |

Reaction of PP890 acid (VIII) and TFX-OH (IX) for 3 hours at 140 °C in the presence of a small amount of zirconium tetrachloride (1 mol%) gave very little conversion (2 %). Addiction of more catalyst (10 mol %) resumed in clean and rapid conversion to the desired tefluthrin product (VII).

The identity of the product was confirmed by GCMS and NMR comparison with authentic material.

Further experimental details:

| Materials | Actual Wt (g) | Strength (%) | 100% Wt. (g) | Mol Wt. | mmol. Wt | Mol. Ratio |
|---|---|---|---|---|---|---|
| TFXOH | 8.0 | 96.6 | 7.8 | 194 | 40 | 1.0 |
| PP890 | 9.8 | 99.5 | 9.7 | 243 | 40 | 1.0 |
| ZrCl₂ | 0.1 | 98 | 0.1 | 233 | 4 | 0.1 |
| Xylene | 100ml | - | - | - | - | - |

### Procedure

A 250ml three neck round bottom flask was fitted with a magnetic flea, thermometer, oil bath, condenser, and Dean & Stark apparatus filled with 3A molecular sieves 8-12 mesh (with 10ml xylene). The system was purged with nitrogen and vented to atmosphere.

TFXOH (8.0g), PP890 (9.8g), xylene (100ml) and ZrCl₄ (0.01g) were charged to the flask. The mixture was heated to reflex (145°C) and held on temperature tor 5hrs. Further ZrCl₄ catalyst (0.09g) was gadded, the liquid in the Dean & Stark receiver replaced with fresh Xylene, and the mixture refluxed for a further 4.5hr.

Reaction was monitored via GC:
Conversion after 1hr (0.1 equivalent catalyst) = 65%
Conversion after 3hr (0.1 equivalent catalyst) = 90%
Conversion after 4.5hr (0.1 equivalent catalyst) = 99%

A portion (20ml) of the readjoumass was washed with water (2x10ml), dried (MgSO₄) and concentrated *in vacuo.* The identity of the product was confirmed by GC, GCMS and NMR.

### Reference Example 2

### Reaction Sequence

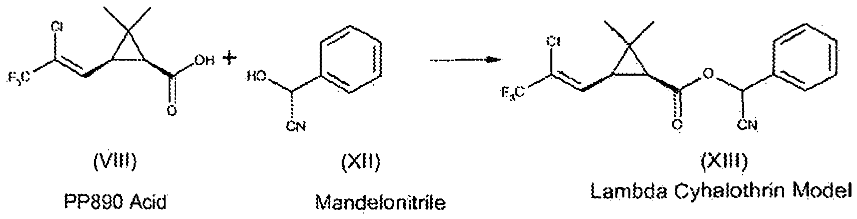

The procedure of Example 1 was repeated varying the sub-strate employed.

| Solvent | Conditions | Time | Catalyst | Conversion |
|---|---|---|---|---|
| Xylene | Reflux, Dean & Stark | 3 hours | None | 2% |
| Xylene | Reflux, Dean & Stark | 3 hours | ZrCl₄ (10 Mol %) | 20% |

The cyanohydrin needed for cyhalothrin is not readily available, and is generated in situ. Madelonitrile is readily available and structurally similar, so this was used as a model for the cyhalothrin system.

Little reaction occurred in the absence of any catalyst (2% conversion after 3 hours at 140 °C.

Significant conversion was achieved in the presence of zirconium tetrachloride catalyst (10 mol %).

### Example 3

### Reaction sequence

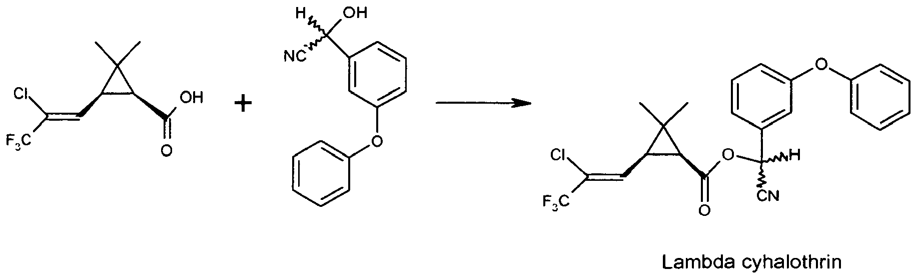

| **Materials** | | **Actual Wt (g)** | **Strength (%)** | **100% Wt. (g)** | **Mol Wt.** | **mmol.** | **Mol. Ratio** |
|---|---|---|---|---|---|---|---|
| 3-phenoxy benzylaldehyde cyandohydrin | | 0.66 | 70 | 0.462 | 225 | 2.05 | 1.0 |
| PP890 | | 0.5 | 100 | 0.498 | 242.5 | 2.05 | 1.0 |
| Diphenylammonium triflilate | | 95 | 0.063 | 0.063 | 309 | 0.205 | 0.1 |
| Xylene | | 20ml | | | | | 62.4 |

A 50ml three neck round bottom flask was fitted with a magnetic flea, thermometer, oil bath, condenser, and Dean & Stark apparatus filled with 3A molecular sieves 8-12 mesh (with 10mol xylene). The system was purged with nitrogen and vented to atmosphere.

3-phenoxy benzylaldehyde cyandohydrin (0.66g), PP890 (0.5g), xylene (20ml) and Diphenylammonium triflilate catalyst (67mg) were charged to the flask. The mixture was heated to reflux (~143 deg C) and held on temperature for 10hrs. The reaction was monitored via GC.

Conversion = 40% (of two cyhalothrin isomers) after 10hr.

GC/MS: The product had same retention time, molecular ion (M⁺ 449) and fragmentation pattern as found with authentic material.

NMR: The NMR data was consistent with that for authentic material.

### Example 4

### Reaction sequence

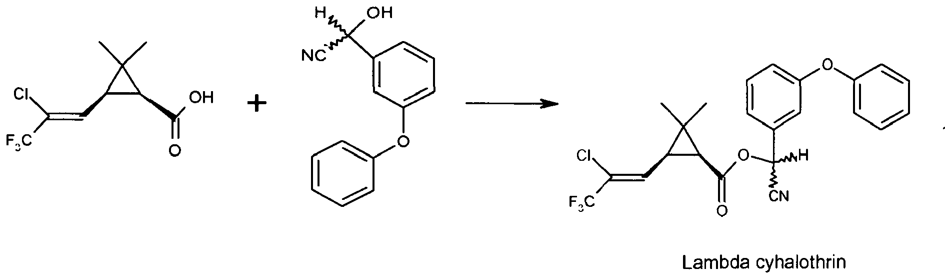

Direct acylation of 3-phenoxy benzylaldehyde cyanohydrin with PP890 was carried out using conditions described in Example 3. A selection of catalysts was screened:

| **Catalyst** | **Solvent** | **Condition** | **Results** |
|---|---|---|---|
| None* | Xylene | Vigorous reflux with Dean & Stark set-up 5.5hr | No reaction. |
| B(OH)₃* | Xylene | Vigorous reflux with Dean & Stark set-up 5.5her | 20% conversion 5% in total of the two cyhalothrin isomers and 15% of two unidentified impurities |
| HfCl₄.2THF* | Xylene | Vigorous reflux with Dean & Stark set-up 5.5her | 20% conversion 10% each of two cyhalothrin isomers |
| Ph₂NH₂⁺OTf | Xylene | Vigorous reflux with Dean & Stark set-up overnight | 46% conversion Total of 37% of the two cyhalothrin isomers and 9% of the two unidentified impurities |

| | | | |
|---|---|---|---|
| * comparative example | | | |

cyhalothrin was successfully formed in the presence of catalyst, and no reaction was observed without catalysis. All three catalysts used gave some conversion to the desired product, but diphenylammonium trifilate was clearly the most effective.

Racemic cyanohydrin and PP890 were used, so the product was formed as a 1:1 mixture of diasteromers:

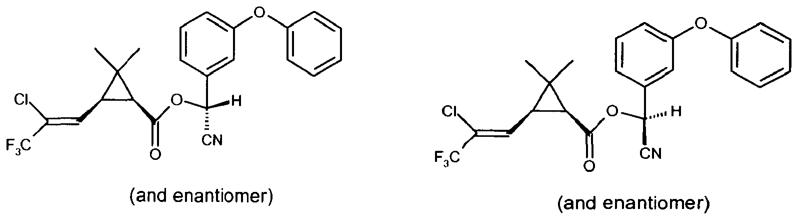

The product was isolated and characterised by NMR and GC/MS. Spectral data for the material were consistent with the desired product.

Two long retention time impurities were seen by GCMS when boric acid and diphenylammonium trifilate were used as catalysts. The two impurities had the same molecular weight suggesting they were isomeric with each other. Their molecular weight (MW 467) was 18 higher than that of the desired product (MW 449). It seems likely therefore that they are hydrated derivatives of the target molecules. E.g. product analogue where the nitrile group has been hydrolysed to an amide:

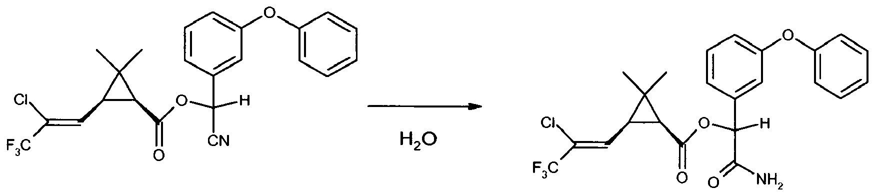

The different behaviour observed between catalysts is thought to be due to differences in the nitrogen purging used. Less efficient purging was used with the boric acid and triflate experiments, which may have allowed some ingress of water (resulting in product hydrolysis).

## Claims

1. A process for the preparation of compounds of formula (III) 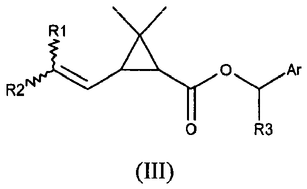 wherein
R1 is hydrogen, alkyl, haloalkyl, or halogen;
R2 is hydrogen, alkyl, haloalkyl, or halogen;
R3 is hydrogen, cyano or alkynyl;
Ar is an optionally substituted aryl or heteroaryl group comprising reacting a carboxylic acid of formula (IV) 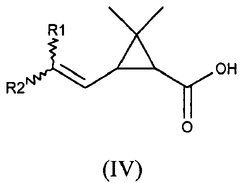 with an alcohol of the formula (V) 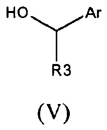 in the presence of a catalyst selected from ammonium salt catalysts.

2. The process according to claim 1, wherein the catalyst is Ph₂NH₂⁺OTf.

3. The process according to either claim 1 or claim 2, wherein R1 and R2 are independently selected from hydrogen, halogen, C1-C4 alkyl and C1-C4 haloalkyl.

4. The process according to any one of claims 1 to 3, wherein R3 is selected from hydrogen, cyano or C3-C4 alkynyl.

5. The process according to any one of claims 1 to 4, wherein Ar is phenyl substituted with one or more groups selected from hydrogen, halogen, hydroxyl, nitro, cyano, C1-C4 alkyl, C1-C4 haloalkyl, and phenoxy.

6. The process according to any preceding claim wherein the carboxylic acid of
formula (IV) has the formula (VIII)

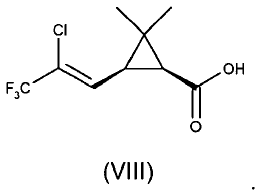

7. The process according to any preceding claim wherein the alcohol of formula (V)
has the formula (IX)

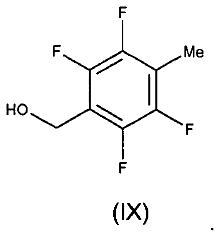

8. The process according to any one of claims 1 to 6 wherein the alcohol of formula (V) has the formula (XI)

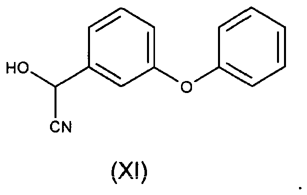

9. The process according to claim 7 for the preparation of tefluthrin (VII)

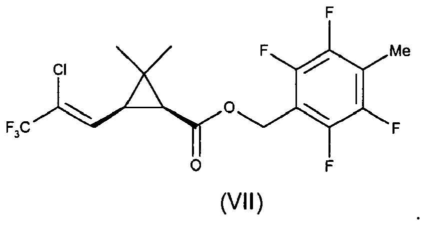

10. The process according to claim 8 for the preparation of lambda cyhalothrin (X)

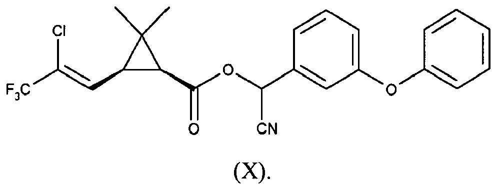

11. The process according to claim 8 for the preparation of gamma cyhalothrin (XIV)

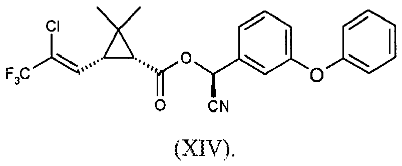

## Patentansprüche

1. Eine Methode für die Bildung der Verbindungen der Formel (III) 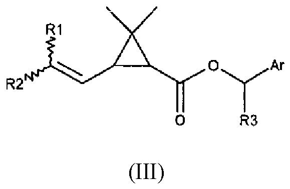 worin
R1 ist Wasserstoff, Alkyl, Haloalkyl, oder Halogen;
R2 ist Wasserstoff, Alkyl, Haloalkyl, oder Halogen;
R3 ist Wasserstoff, Cyan, oder Alkenyl,
Ar ist eine wahlweise substituierte Aryl- oder Heteroarylgruppe und umfasst die Reaktion einer Carboxylsäure der Formel (IV) 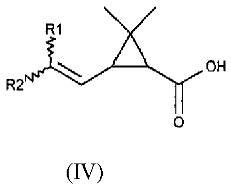 mit einem Alkohol der Formel (V) 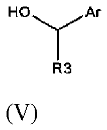 im Beisein eines von Ammoniumsalzkatalysatoren gewählten Katalysators.

2. Die Methode gemäß Anspruch 1, worin der Katalysator Ph₂NH₂⁺OTf ist.

3. Die Methode gemäß entweder Anspruch 1 oder Anspruch 2, worin R1 and R2 vom Wasserstoff, Halogen, C1-C4-Alkyl und C1-C4-Haloalkyl unabhängig gewählt werden.

4. Die Methode gemäß einem etwaigen Anspruch 1 bis 3, worin R3 vom Wasserstoff, Cyan, oder C3-C4-Alkinyl gewählt wird.

5. Die Methode gemäß einem etwaigen Anspruch 1 bis 4, worin Ar ist Phenyl substituiert durch eine oder mehrere Gruppen gewählt aus Wasserstoff, Halogen, Hydroxyl, Nitro, Cyan, C1-C4-Alkyl, C1-C4-Haloalkyl und Phenoxy.

6. Die Methode gemäß einem etwaigen vorangehenden Anspruch, worin die Carboxylsäure der
Formel (IV) die Formel (VIII) aufweist

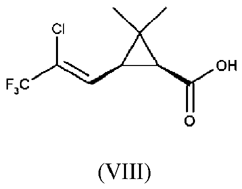

7. Die Methode gemäß einem etwaigen vorangehenden Anspruch, worin der Alkohol der Formel (V) die Formel (IX) aufweist

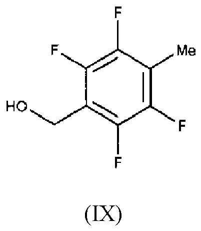

8. Die Methode gemäß einem etwaigen Anspruch 1 bis 6, worin der Alkohol der Formel (V) die Formel (XI) aufweist

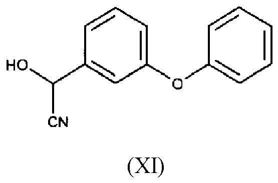

9. Die Methode für die Herstellung vom Tefluthrin (VII) gemäß Anspruch 7

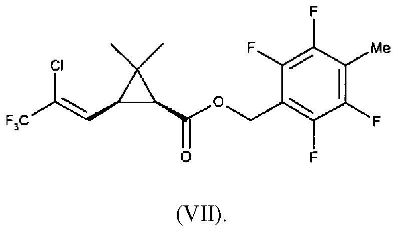

10. Die Methode für die Herstellung vom Lambda-Cyhalothrin (X) gemäß Anspruch 8

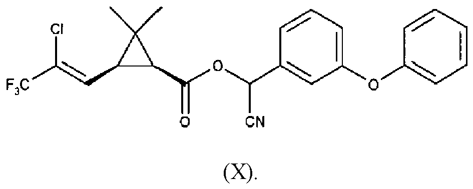

11. Die Methode für die Herstellung vom Gamma-Cyhalothrin (XIV) gemäß Anspruch 8

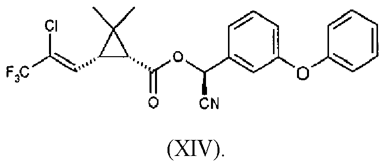

## Revendications

1. Procédé de préparation de composés selon la formule (III) 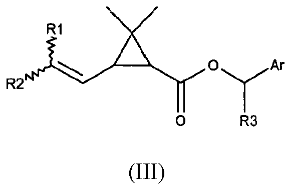 dans lequel
R1 est un groupe hydrogène, alkyle, halogénure d'alkyle ou halogène ; R2 est un groupe hydrogène, alkyle, halogénure d'alkyle ou halogène ; R3 est un groupe hydrogène, cyano ou alcyne ;
Ar est un groupe aryle ou hétéroaryle éventuellement substitué, comprenant la réaction d'un acide carboxylique selon la formule (IV) 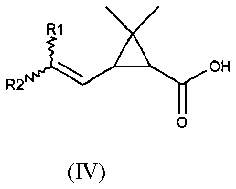 avec un alcool selon la formule (V) 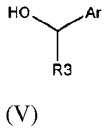 en présence d'un catalyseur sélectionné parmi des catalyseurs à base de sel d'ammonium.

2. Procédé selon la revendication 1, dans lequel le catalyseur est Ph₂NH2+^{O}Tf.

3. Procédé selon soit la revendication 1, soit la revendication 2, dans lequel R1 et R2 sont sélectionnés indépendamment parmi les groupes hydrogène, halogène, alkyle C1-C4 et halogénure d'alkyle C1-C4.

4. Procédé selon l'une quelconque des revendications 1 à 3, dans lequel R3 est sélectionné parmi les groupes hydrogène, cyano ou alcyne C3-C4.

5. Procédé selon l'une quelconque des revendications 1 à 4, dans lequel Ar est un phényle substitué par un ou plusieurs groupes sélectionnés parmi les groupes hydrogène, halogène, hydroxyle, nitro, cyano, alkyle C1-C4, halogénure d'alkyle C1-C4 et phénoxy.

6. Procédé selon l'une quelconque des revendications précédentes, dans lequel l'acide carboxylique selon la formule (IV) répond à la formule (VIII)

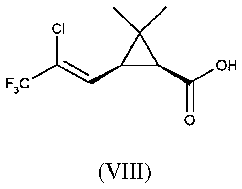

7. Procédé selon l'une quelconque des revendications précédentes, dans lequel l'alcool selon la formule (V) répond à la formule (IX)

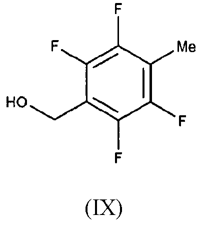

8. Procédé selon l'une quelconque des revendications 1 à 6, dans lequel l'alcool selon la formule (V) répond à la formule (XI)

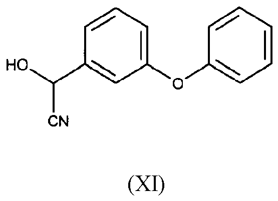

9. Procédé selon la revendication 7 pour préparer de la téfluthrine (VII)

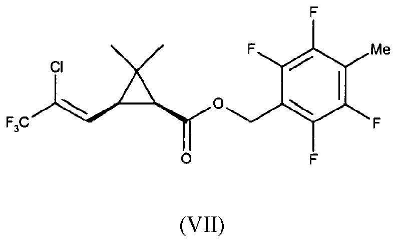

10. Procédé selon la revendication 8 pour préparer de la lambda-cyhalothrine (X)

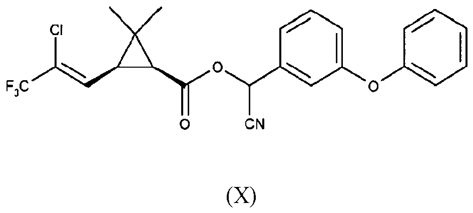

11. Procédé selon la revendication 8 pour préparer de la gamma-cyhalothrine (XIV)
